# EUROPEAN PATENT APPLICATION

(11) **EP 4 769 419 A1**
(43) Date of publication of application: **01.07.2026**
(21) Application number: 24856797.6
(22) Date of filing: 20.08.2024
(51) Int. Cl.: G16C 20/70, G16C 20/30, G16C 20/90

(54) **APPARATUS AND METHOD FOR GENERATING FINGERPRINT**

(30) Priority: 24.08.2023 KR 20230111298
(71) Applicant: Hanwha Solutions Corporation, Jung-gu Seoul 04541 (KR)
(72) Inventor: PARK, Chan Hyung, Seoul 04541 (KR); SAGONG, Kil, Daejeon 34128 (KR); LEE, Won Jong, Daejeon 34128 (KR); LIM, Geunsik, Daejeon 34128 (KR); KIM, Dong Wook, Daejeon 34128 (KR); WON, Bokyoung, Seoul 04541 (KR); KIM, Daeshik, Seoul 04541 (KR); EUM, Soomin, Seoul 04541 (KR)
(74) Representative: Berggren Oy
(86) International application number: PCT/KR2024/012371
(87) International publication number: WO 2025/042186

(57) **Abstract**

An apparatus for generating a fingerprint, according to an embodiment of the present invention, comprises: a fingerprint conversion module that converts chemical formulas of a plurality of chemical substances into fingerprints, respectively; a fingerprint mixing module that multiplies the respective converted fingerprints by mixing coefficient and adds the fingerprints; and a fingerprint learning module that learns the added fingerprints to generate a final fingerprint. Therefore, a fingerprint suitable for an experimental condition or a physical property to be predicted can be generated by means of learning.

## Description

### TECHNICAL FIELD

The present invention relates to a fingerprint generation technology for predicting the physical properties of a chemical formula.

### BACKGROUND ART

In order to predict physical properties, researchers have been using methods to learn the relationship between the chemical formula and physical properties of chemical substances, and to predict the physical properties of new substances based on this learning.

However, since artificial intelligence cannot understand chemical formulas themselves, in order to learn chemical formulas, chemical formulas are converted into numerical data and used to learn the relationship between chemical formulas and physical properties.

Fingerprints can be used as structured data consisting of numbers. However, it is difficult to secure sufficient fingerprint samples, and fingerprints have the problem of not reflecting experimental conditions or properties to be predicted.

The inventors of the present invention have made research efforts to overcome the limitations of the fingerprint generation method of the conventional technology. After much effort, the inventors of the present invention have completed the present invention to provide a method capable of generating different fingerprints for the same compound by reflecting experimental conditions and the characteristics of a substance to be predicted in the fingerprint generation.

### DISCLOSURE

### TECHNICAL PROBLEM

An object of the present invention is to provide an apparatus and method for generating a fingerprint that are capable of generating a suitable fingerprint according to a physical property to be predicted or experimental conditions.

Meanwhile, other unspecified objects of the present invention will be additionally considered within a scope that can be easily inferred from the following detailed description and its effects.

### TECHNICAL SOLUTION

The apparatus for generating a fingerprint according to an embodiment of the present invention may include a fingerprint conversion module configured to convert chemical formulas of a plurality of chemical substances into fingerprints, respectively; a fingerprint mixing module configured to sum the converted fingerprints after multiplying each of the converted fingerprints by a corresponding mixing coefficient; and a fingerprint learning module configured to generate a final fingerprint by the added fingerprints.

The fingerprint conversion module may convert the same chemical formula into a plurality of fingerprints by a plurality of fingerprint conversion algorithms.

The fingerprint mixing module may add a constant according to experimental conditions to fingerprints that are added up by multiplying the mixing coefficient and transfer to the fingerprint learning module.

The fingerprint learning module may be an embedding layer.

The fingerprint learning module may use results of learning the added fingerprints differently depending on a physical property to be predicted.

The method for generating a fingerprint according to another embodiment of the present invention may include converting chemical formulas of a plurality of chemical substances into fingerprints, respectively; adding up fingerprints obtained by multiplying each of the converted fingerprints by different mixing coefficients for each of the plurality of chemical substances; and generating a final fingerprint by the added fingerprints.

The converting into fingerprints may convert the same chemical formula into a plurality of fingerprints by a plurality of fingerprint conversion algorithms.

The adding up may add a constant according to experimental conditions to fingerprints that are added up by multiplying the mixing coefficient.

The generating a final fingerprint may learn the added fingerprints by an embedding layer.

The generating a final fingerprint may use results of learning the added fingerprints differently depending on a physical property to be predicted.

### ADVANTAGEOUS EFFECT

According to the present invention, since it is possible to predict the physical properties of a required substance by generating a suitable fingerprint through learning according to the physical properties to be predicted or experimental conditions, it has is an advantage in that the experimental period and resources consumed for developing new materials can be saved.

Meanwhile, it is added that even if an effect is not explicitly mentioned herein, the effects and its provisional effects described in the following specification expected by the technical features of the present invention are treated as described in the specification of the present invention.

### DESCRIPTION OF THE DRAWINGS

FIG. 1 is a diagram showing the overall structure of a physical property prediction system according to any preferred embodiment of the present invention.
FIG. 2 is a schematic structural diagram of an apparatus for generating a fingerprint according to any preferred embodiment of the present invention.
FIG. 3 is a more detailed structural diagram of a fingerprint conversion module according to any preferred embodiment of the present invention.
FIG. 4 is a more detailed structural diagram of a fingerprint mixing module according to any preferred embodiment of the present invention.
FIG. 5 shows an example of a fingerprint generation process of an apparatus for generating a fingerprint according to any preferred embodiment of the present invention.
FIG. 6 is a schematic flowchart of a method for generating a fingerprint according to another preferred embodiment of the present invention.
   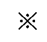 It is stated that the attached drawings are provided as references to help understand the technical concept of the present invention, and the scope of the rights of the present invention is not limited thereby.

### MODES FOR INVENTION

Hereinafter, preferred embodiments of the present invention will be described in detail with reference to the accompanying drawings. The detailed description to be disclosed below together with the accompanying drawings is intended to describe exemplary embodiments of the present invention, and is not intended to represent the only embodiments in which the present invention may be practiced. In order to clearly describe the present invention in the drawings, parts that are not related to the description may be omitted, and the same reference numerals may be used for the same or similar components throughout the specification.

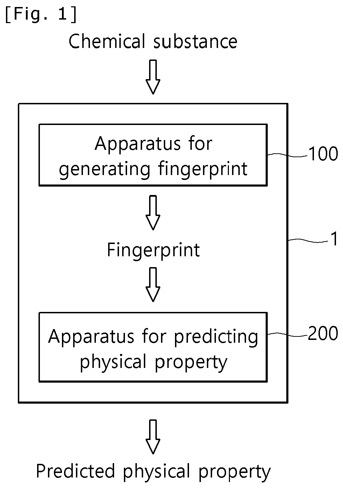

FIG. 1 is a diagram showing the overall structure of a physical property prediction system according to a preferred embodiment of the present invention.

An artificial intelligence-based material property prediction system 1 may be used to predict material properties for the development of new materials.

A property prediction system 1 may include an apparatus for generating a fingerprint 100 and an apparatus for predicting a physical property 200.

Since artificial intelligence models do not understand the chemical formula of a chemical substance on its own, it must be converted into a numerical fingerprint and input. Through this, an artificial intelligence-based apparatus for generating a fingerprint 100 and an apparatus for predicting a physical property 200 may learn, and the learned results may be used to predict the physical property.

When the chemical formula of a chemical substance whose physical properties are to be predicted is input, the apparatus for generating a fingerprint 100 generates a fingerprint by artificial intelligence (machine learning) learned from the fingerprint itself.

In the conventional technology, this fingerprint is generated singly for each chemical substance, and the number of chemical substances that can be utilized for learning, that is, chemical substances that can generate fingerprints, is limited, and thus, there was a problem in that general databases (public data in the chemical field - PubChem, Catalysis-hub, Materials project, etc.) could not be utilized.

Therefore, the apparatus for generating a fingerprint 100 according to the present invention has the purpose of enabling the prediction of physical properties even if they are not previously learned fingerprints by learning fingerprints differently depending on experimental conditions or physical properties to be predicted, even for the same chemical substance.

The chemical formula of a chemical substance is converted into a fingerprint by a fingerprint generating apparatus 100, and this is input into an apparatus for predicting a physical property 200 to predict the physical properties of the chemical substance.

The apparatus for predicting a physical property 200 may be composed of an experimental data learning module, a physical property prediction module and a screening module.

The experimental data learning module additionally learns experimental data from a pre-trained model for physical property prediction, thereby generating a final model for physical property prediction of candidate materials. In other words, by using data obtained through actual experiments for additional learning of the physical property prediction model, the physical property prediction model may be supplemented by using artificial intelligence.

The physical property prediction module uses the learned physical property prediction model to predict the physical properties of candidate chemicals. In this case, physical property prediction is performed by using a fingerprint generated by an apparatus for generating a fingerprint 100.

The screening module may screen candidate substances that are suitable for the target properties and constraints by synthesizing the physical properties predicted by the property prediction module, and may recommend final candidate substances that are close to the required physical properties accordingly.

The final candidate substances selected through screening are judged to have the required physical properties through actual experiments, and the experimental results are used for additional learning by the experimental data learning module, thereby creating a more accurate physical property prediction model.

In addition, there is an advantage in that the cost and time required for the experiment can be saved by conducting the actual experiment only with candidate substances screened by the apparatus for predicting a physical property 200.

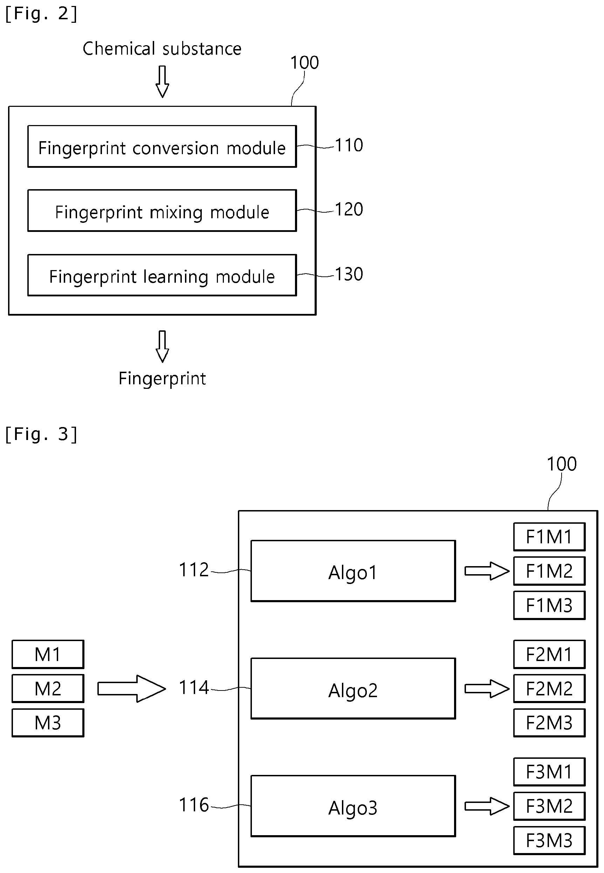

FIG. 2 is a schematic structural diagram of an apparatus for generating a fingerprint 100 of such a physical property prediction system 1.

The apparatus for generating a fingerprint 100 according to the present invention may be composed of a fingerprint conversion module 100, a fingerprint mixing module 120 and a fingerprint learning module 130.

When the chemical formulas of a plurality of chemical substances are input, each chemical formula is converted into a fingerprint by the fingerprint conversion module 110. The fingerprint mixing module 120 multiplies each fingerprint by a weight, adds them up and then connects a constant according to experimental conditions to generate a new fingerprint.

In this case, even if the chemical formula is the same, it is converted into a plurality of fingerprints by the fingerprint conversion module 110, and the fingerprint that reflects the experimental conditions by multiplying the weights and adding up is generated as a final fingerprint by the fingerprint learning module 130.

FIG. 3 is a more detailed structural diagram of a fingerprint conversion module 110 according to a preferred embodiment of the present invention.

The fingerprint conversion module 110 according to the present invention has a difference from conventional fingerprint conversion methods in that it uses a plurality of algorithms 112, 114, 116.

Chemical formulas M1, M2, M3 for a plurality of chemical substances are converted into fingerprints by a first algorithm Algo1, 112, a second algorithm Algo2, 114 and a third algorithm Algo3, 116, respectively.

That is, M1, M2 and M3 are converted into fingerprints of F1M1, F1M2 and F1M3, respectively, by the first algorithm 112, into F2M1, F2M2 and F2M3 by the second algorithm 114, and into F3M1, F3M2 and F3M3 by the third algorithm 116.

The plurality of algorithms may include, but are not limited to, algorithms such as TOKENIZER, ATOM-TRIPLET, QSPR, SMILES Pair Encoding (SPE), Morphological descriptor, E-state, Joback Method and the like

TOKENIZER is a method that converts each part of a chemical substance into a fingerprint based on a newly defined chemical formula that reflects the structural characteristics of the target chemical substance for physical property prediction, and has the characteristic of being able to reflect the structural characteristics of a chemical substance in the fingerprint.

ATOM-TRIPLET converts chemical structures into fingerprints based on three consecutive atomic groups. Therefore, it has the characteristic of being able to reflect the atomic-level microscopic structure of a chemical substance into a fingerprint.

QPSR is an algorithm that quantitatively describes the relationship between the overall structure and properties of a chemical substance. Therefore, the macroscopic properties of a chemical substance may be reflected in the fingerprint.

Fingerprints converted by various algorithms are transferred to the following fingerprint mixing module 120 and added up.

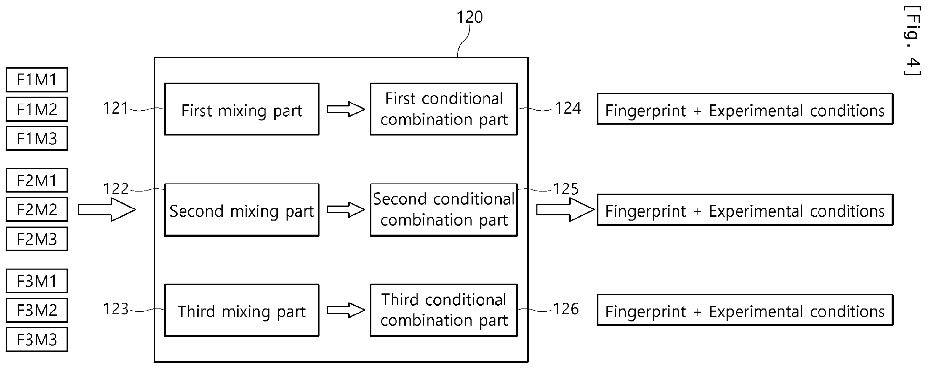

FIG. 4 is a more detailed structural diagram of a fingerprint mixing module 120 according to a preferred embodiment of the present invention.

The fingerprint mixing module 120 may be composed of mixing parts 121, 122, 123 and combination parts 124, 125, 126.

The mixing parts 121, 122, 123 multiply fingerprints generated according to each algorithm by different weights (ratios) and then adds them up to generate one fingerprint for each algorithm.

For example, fingerprints F1M1, F1M2, F1M3 generated by the first algorithm 112 are each multiplied by different weights by the first mixing part 121 and added up together to generate one fingerprint.

In another embodiment, a method may be used in which fingerprints generated by chemical formulas are added up rather than adding up fingerprints by each algorithm.

To this end, the mixing parts 121, 122, 123 may generate a single fingerprint by multiplying the fingerprints converted to the same chemical formula among the fingerprints generated according to each algorithm by the weights and adding them up together.

For example, for a substance M1, fingerprints F1M1, F2M1, F3M1 generated by three algorithms are multiplied by different weights and added up together by the first mixing part 121 to generate one fingerprint.

A constant according to experimental conditions may be combined with the fingerprint thus added up by the combination parts 124, 125, 126. That is, a new fingerprint is generated by adding experimental conditions such as temperature or pressure to the previously created fingerprint.

Accordingly, a plurality of chemical formulas are generated as one fingerprint for each algorithm or one fingerprint for each chemical formula by the fingerprint mixing module 120 and transmitted to the fingerprint learning module 130.

The fingerprint learning module 130 learns from fingerprints generated by the fingerprint mixing module 120, and generates a final fingerprint based on the learning results.

To this end, the fingerprint learning module 130 may be composed of an embedding layer, but is not limited thereto, and various learning algorithms such as a fully connected layer (FC), word2vec, glove and Fasttext may be used.

Fingerprints for each algorithm are generated into a final fingerprint by the fingerprint learning module 130.

In this case, the fingerprint learning module 130 may learn differently depending on the physical properties to be predicted, such as viscosity prediction, density prediction and the like. Therefore, even for the same chemical substance, the fingerprint finally generated by the fingerprint learning module 130 may show different results depending on the physical properties.

The fingerprint generated in this way is transmitted to an apparatus for predicting a physical property 200 and used to predict whether it has the desired physical property.

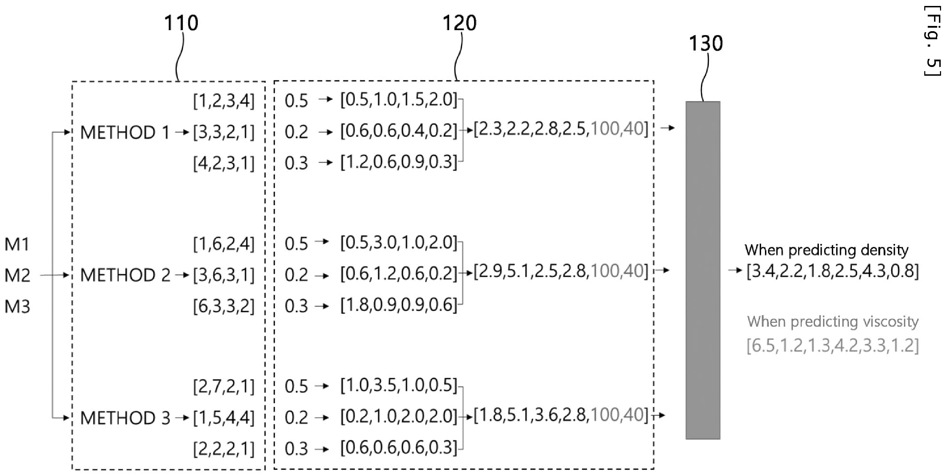

FIG. 5 shows an example of a fingerprint generation process of an apparatus for generating a fingerprint 100 according to a preferred embodiment of the present invention.

When the chemical formulas M1, M2, M3 of three chemical substances are input into the fingerprint conversion module 110, M1, M2 and M3 are converted into different fingerprints by each algorithm (METHOD1, METHOD2, METHOD3).

For example, a chemical substance called M1 was converted to [1, 2, 3, 4] by the first algorithm, to [1, 6, 2, 4] by the second algorithm, and to [1, 6, 2, 4] by the third algorithm.

Next, in the fingerprint mixing module 120, different weights are multiplied for each chemical formula, that is, a weight of 0.5 for M1, 0.2 for M2 and 0.3 for M3, and the results are added up, and a constant according to the experimental conditions is combined.

For example, the three fingerprints generated by the first algorithm are multiplied by their respective weights and then added up together to generate a fingerprint called [2.3,2.2,2.8,2.5], and a constant according to the experimental conditions is combined to generate a fingerprint called [2.3,2.2,2.8,2.5,100,40].

In this way, the fingerprint learning module 130 learns from the fingerprints themselves by combining the experimental conditions by adding up for each algorithm, and it can be confirmed that different final fingerprints are generated by the fingerprint learning module 130 for different physical properties such as density or viscosity.

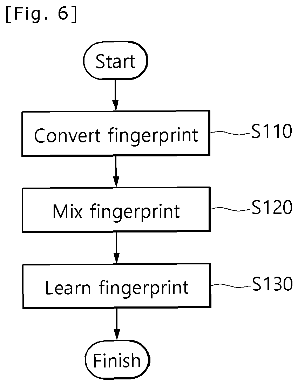

FIG. 6 is a schematic flowchart of a method for generating a fingerprint according to another preferred embodiment of the present invention.

The method for generating a fingerprint according to the present invention may be performed by a controller including one or more processors and a memory, or an apparatus for generating a fingerprint including a controller.

Chemical formulas for a plurality of chemical substances whose physical properties are to be predicted are first converted into fingerprints S110.

In this case, the chemical formulas for a plurality of chemical substances may be converted into fingerprints by a plurality of algorithms. For example, if three conversion algorithms are used to convert the chemical formulas of three substances into fingerprints, a total of nine fingerprints are generated.

The plurality of algorithms may include, but are not limited to, algorithms such as TOKENIZER, ATOM-TRIPLET, QSPR and the like.

A more detailed process is as described above by the fingerprint conversion module 110.

A plurality of fingerprints generated in this way are multiplied by different weights (ratios) for the fingerprints generated according to each algorithm, then added up together, and a constant according to experimental conditions is combined to undergo a fingerprint mixing step to generate one fingerprint for each algorithm S120.

The fingerprints generated for each algorithm are generated as a final fingerprint based on the pre-learned fingerprint learning results S130.

Fingerprint learning may be performed by an embedding layer, but is not limited thereto.

Fingerprint learning is learned differently depending on the physical property to be predicted, such as viscosity prediction, density prediction and the like, and therefore, even for the same chemical substance, the final generated fingerprint may show different results depending on the physical property.

The final generated fingerprint is used to predict the physical properties of the chemical substance by an artificial intelligence-based apparatus for predicting a property prediction.

In this way, according to the apparatus and method for generating a fingerprint according to the present invention, rather than a uniform fingerprint depending on the chemical substance, different fingerprints may be generated depending on the physical properties to be predicted or experimental conditions even for the same chemical substance, and thus, it has an effect of being able to generate a fingerprint that is more suitable for the prediction of a physical property through learning.

Although the detailed description of the present invention has described specific embodiments, it is apparent that various modifications are possible without departing from the scope of the present invention. Therefore, the scope of the present invention is not limited to the described embodiments, but should be determined by the claims and equivalents of the claims.

## Claims

1. An apparatus for generating a fingerprint that comprises one or more processors and a memory, the apparatus comprising:
a fingerprint conversion module configured to convert chemical formulas of a plurality of chemical substances into fingerprints, respectively;
a fingerprint mixing module configured to sum the converted fingerprints after multiplying each of the converted fingerprints by a corresponding mixing coefficient; and
a fingerprint learning module configured to generate a final fingerprint by the added fingerprints.

2. The apparatus of claim 1, wherein the fingerprint conversion module converts the same chemical formula into a plurality of fingerprints by a plurality of fingerprint conversion algorithms.

3. The apparatus of claim 1, wherein the fingerprint mixing module adds a constant according to experimental conditions to fingerprints that are added up by multiplying the mixing coefficient and transfers to the fingerprint learning module.

4. The apparatus of claim 1, wherein the fingerprint learning module is an embedding layer.

5. The apparatus of claim 1, wherein the fingerprint learning module uses results of learning the added fingerprints differently depending on a physical property to be predicted.

6. A method for generating a fingerprint that is performed by a controller comprising one or more processors and a memory, the method comprising:
converting chemical formulas of a plurality of chemical substances into fingerprints, respectively;
adding up fingerprints obtained by multiplying each of the converted fingerprints by different mixing coefficients for each of the plurality of chemical substances; and
generating a final fingerprint by the added fingerprints.

7. The method of claim 6, wherein the converting into fingerprints converts the same chemical formula into a plurality of fingerprints by a plurality of fingerprint conversion algorithms.

8. The method of claim 6, wherein the adding up adds a constant according to experimental conditions to fingerprints that are added up by multiplying the mixing coefficient.

9. The method of claim 6, wherein the generating a final fingerprint learns the added fingerprints by an embedding layer.

10. The method of claim 9, wherein the generating a final fingerprint uses results of learning the added fingerprints differently depending on a physical property to be predicted.
